# EUROPEAN PATENT SPECIFICATION

(11) **EP 3 972 509 B1**
(45) Date of publication and mention of the grant of the patent: **10.09.2025**
(21) Application number: 20753809.1
(22) Date of filing: 31.07.2020
(51) Int. Cl.: A61B 18/14, A61B 17/00

(54) **LESION PREDICTING FLEX TIP**
VERLETZUNGSVORHERSAGENDE BIEGSAME SPITZE
POINTE FLEXIBLE DE PRÉDICTION DE LÉSION

(30) Priority: 02.08.2019 US 201962882207 P
(43) Date of publication of application: 30.03.2022
(73) Proprietor: St. Jude Medical, Cardiology Division, Inc., St. Paul MN 55117 (US)
(72) Inventor: OLIVERIUS, Andrew, Eagan, Minnesota 55122 (US); MARASS, Timothy, Minneapolis, Minnesota 55419 (US); GILBERT, Therese, St. Paul, Minnesota 55116 (US)
(74) Representative: Mathys & Squire
(86) International application number: PCT/IB2020/057294
(87) International publication number: WO 2021/024140

(56) References cited:
- EP-A2- 2 335 632
- WO-A1-2013/101923
- WO-A1-2016/065337
- WO-A1-2016/112196
- US-A- 5 545 193
- US-A- 6 033 403
- US-A1- 2010 042 098
- US-B2- 9 033 976

## Description

### BACKGROUND

### a. Field

The instant disclosure relates generally to a tip electrode for delivering and receiving energy and delivering irrigant to the tip electrode and catheter tip assemblies incorporating such a tip electrode.

### b. Background Art

US 2010/0042098 A1 discloses systems and methods for creating a lesion using a transjugular approach. WO 2016/112196 A1 discloses an ablation catheter with electrodes. US 6,033,403 A discloses a long electrode catheter system and methods thereof. US 5,545,193 A discloses helically wound radio-frequency emitting electrodes for creating lesions in a body tissue. EP 2 335 632 A2 discloses a catheter with a helical electrode. US 9,033,976 B2 discloses a modification of airways by application of energy. WO2016065337 discloses a wire like electrode assembly that can wrap around a distal tip electrode. WO2013101923 discloses flexible tip electrodes with ring electrodes.

Electrophysiology catheters are used in a variety of diagnostic, therapeutic, and/or mapping and ablative procedures to diagnose and/or correct conditions such as atrial arrhythmias, including for example, ectopic atrial tachycardia, atrial fibrillation, and atrial flutter. Arrhythmias can create a variety of conditions including irregular heart rates, loss of synchronous atrioventricular contractions and stasis of blood flow in a chamber of a heart which can lead to a variety of symptomatic and asymptomatic ailments and even death.

Typically, a catheter is deployed and manipulated through a patient's vasculature to the intended site, for example, a site within a patient's heart or a chamber or vein thereof. The catheter carries one or more electrodes that can be used for cardiac mapping or diagnosis, ablation and/or other therapy delivery modes, or both, for example. Once at the intended site, treatment can include, for example, radio frequency (RF) ablation, cryoablation, laser ablation, chemical ablation, high-intensity focused ultrasound-based ablation, microwave ablation, and/or other ablation treatments. The catheter imparts ablative energy to cardiac tissue to create one or more lesions in the cardiac tissue and oftentimes a contiguous or linear and transmural lesion. This lesion disrupts undesirable cardiac activation pathways and thereby limits, corrals, or prevents errant conduction signals that can form the basis for arrhythmias.

To position a catheter within the body at a desired site, some type of navigation must be used, such as using mechanical steering features incorporated into the catheter (or an introducer sheath). In some examples, medical personnel may manually manipulate and/or operate the catheter using the mechanical steering features.

In order to facilitate the advancement of catheters through a patient's vasculature, the simultaneous application of torque at the proximal end of the catheter and the ability to selectively deflect the distal tip of the catheter in a desired direction can permit medical personnel to adjust the direction of advancement of the distal end of the catheter and to position the distal portion of the catheter during an electrophysiological procedure. The proximal end of the catheter can be manipulated to guide the catheter through a patient's vasculature. The distal tip can be deflected by a pull wire attached at the distal end of the catheter that extends to a control handle that controls the application of tension on the pull wire.

A medical procedure in which an electrophysiology catheter is used includes a first diagnostic catheter deployed through a patient's vasculature to a patient's heart or a chamber or vein thereof. An electrophysiology catheter that carries one or more electrodes can be used for cardiac mapping or diagnosis, ablation and/or other therapy delivery modes, or both. Once at the intended site, treatment can include radio frequency (RF) ablation, cryoablation, laser ablation, chemical ablation, high-intensity focused ultrasound-based ablation, microwave ablation, etc. An electrophysiology catheter imparts ablative energy to cardiac tissue to create one or more lesions in the cardiac tissue and oftentimes a contiguous or linear and transmural lesion. This lesion disrupts undesirable cardiac activation pathways and thereby limits, corrals, or prevents stray errant conduction signals that can form the basis for arrhythmias.

Because RF ablation can generate significant heat, which if not controlled can result in excessive tissue damages, such as steam pop, tissue charring, and the like, it can be desirable to monitor the temperature of ablation electrode assemblies. It can also be desirable to include a mechanism to irrigate the ablation electrode assemblies and/or targeted areas in a patient's body with biocompatible fluids, such as saline solution. The use of irrigated ablation electrode assemblies can also prevent the formation of soft thrombus and/or blood coagulation, as well as enable deeper and/or greater volume lesions as compared to conventional, non-irrigated catheters at identical power settings.

The foregoing discussion is intended only to illustrate the present field and should not be taken as a disavowal of claim scope.

### BRIEF SUMMARY

The invention is defined by the appended claims. In one embodiment, a catheter tip electrode can comprise a flexible electrode structure configured to flex upon application of an external force, a distal end portion adjacent the flexible electrode structure and defining a distal end, and a wire electrode.

In another embodiment, a catheter assembly can comprise a catheter body, a tip electrode, and a wire electrode. The tip electrode can be coupled to a distal end of the catheter body.

In yet another embodiment, a catheter tip electrode can comprise a flexible electrode structure configured to flex upon application of an external force, a distal end portion adjacent the flexible electrode structure and defining a distal end, a first wire electrode, and a second wire electrode.

The foregoing and other aspects, features, details, utilities, and advantages of the present disclosure will be apparent from reading the following description and claims, and from reviewing the accompanying drawings.

### BRIEF DESCRIPTION OF THE DRAWINGS

FIG. 1 is a schematic view of a catheter incorporating a deflectable catheter shaft section in accordance with an embodiment.
FIG. 2 is a schematic view of a catheter incorporating a deflectable catheter shaft section in accordance with another embodiment.
FIG. 3 is a fragmentary view of a deflectable catheter shaft section and an intermediate catheter shaft section.
FIG. 4 is a cross-sectional view taken along line 4-4 of FIG. 3.
FIG. 5 is an end view taken in the direction of line 5-5 of FIG. 4.
FIG. 6 is a side view of one embodiment of a catheter according to the disclosure.
FIG. 7 is an isometric view of one embodiment of a tip assembly according to the disclosure.
FIG. 8 is an isometric side view of one embodiment of an electrode cap.
FIG. 9 is an isometric side view of one embodiment of a partial tip electrode.
FIG. 10 is an isometric side view of an embodiment of an electrode cap.
FIG. 11 is a partial side view of a distal portion of a tip electrode.
FIG. 12 is a partial side view of another embodiment of a tip electrode.
FIG. 13 is a partial side view of a catheter.
FIG. 14 is a partial side view of another embodiment of a catheter.
FIG. 15 is a side view of another embodiment of a wire electrode disposed on a catheter shaft.
FIG. 16 is a cross-sectional view of a catheter shaft.
FIGS. 17A and 17B are a side view and an end view of one embodiment of a tip electrode.
FIG. 18 is a side view of one embodiment of a tip electrode with a wire electrode contacting a tissue.
FIG. 19 is side view of another embodiment of a tip electrode with a plurality of wire electrodes.
FIGS. 20 and 21 are isometric views of two embodiments of a wire electrode.
FIGS. 22 and 23 are close-up, cross-sectional views of two embodiments of an electrode with a channel for a wire electrode.

### DETAILED DESCRIPTION OF THE DISCLOSURE

Embodiments are described herein of various apparatuses, systems, and/or methods. Numerous specific details are set forth to provide a thorough understanding of the overall structure, function, manufacture, and use of the embodiments as described in the specification and illustrated in the accompanying drawings. It will be understood by those skilled in the art, however, that the embodiments may be practiced without such specific details. In other instances, well-known operations, components, and elements have not been described in detail so as not to obscure the embodiments described in the specification. Those of ordinary skill in the art will understand that the embodiments described and illustrated herein are non-limiting examples, and thus it can be appreciated that the specific structural and functional details disclosed herein may be representative and do not necessarily limit the scope of the embodiments, the scope of which is defined solely by the appended claims.

Reference throughout the specification to "various embodiments," "some embodiments," "one embodiment," or "an embodiment", or the like, means that a particular feature, structure, or characteristic described in connection with the embodiment(s) is included in at least one embodiment. Thus, appearances of the phrases "in various embodiments," "in some embodiments," "in one embodiment," or "in an embodiment," or the like, in places throughout the specification, are not necessarily all referring to the same embodiment. Furthermore, the particular features, structures, or characteristics may be combined in any suitable manner in one or more embodiments. Thus, the particular features, structures, or characteristics illustrated or described in connection with one embodiment may be combined, in whole or in part, with the features, structures, or characteristics of one or more other embodiments without limitation given that such combination is not illogical or non-functional.

It will be appreciated that the terms "proximal" and "distal" may be used throughout the specification with reference to a clinician manipulating one end of an instrument used to treat a patient. The term "proximal" refers to the portion of the instrument closest to the clinician and the term "distal" refers to the portion located furthest from the clinician. It will be further appreciated that for conciseness and clarity, spatial terms such as "vertical," "horizontal," "up," and "down" may be used herein with respect to the illustrated embodiments. However, medical instruments may be used in many orientations and positions, and these terms are not intended to be limiting and absolute.

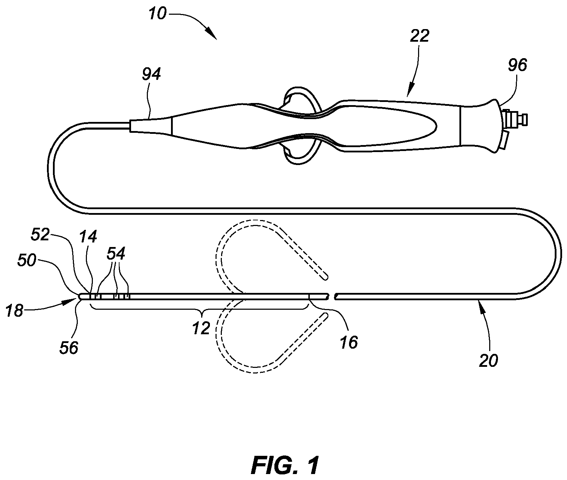

FIG. 1 generally illustrates a deflectable electrophysiology catheter 10 that comprises a deflectable catheter shaft section 12 in accordance with an embodiment. Deflectable catheter shaft section 12 comprises an elongated body having a distal end 14 and a proximal end 16. In its most general form, catheter 10 further comprises a tip assembly 18 located at the distal end 14 of the deflectable catheter shaft section 12, a proximal catheter shaft section 20 located at the proximal end 16 of the deflectable catheter shaft section 12, and a handle assembly 22. Catheter 10 may be used in any number of diagnostic and therapeutic applications, such as the recording of electrograms in the heart, the performance of a cardiac ablation procedure, and other similar applications/procedures. Accordingly, one of ordinary skill in the art will recognize and appreciate that the inventive deflectable catheter shaft section and method of manufacturing the same can be used in any number of diagnostic and therapeutic applications.

Still referring to FIG. 1, deflectable catheter shaft section 12 is disposed between the tip assembly 18 and the proximal catheter shaft section 20. The length and diameter of the deflectable catheter shaft section 12 can vary according to the application. Generally, the length of the deflectable catheter shaft section 12 can range from about 2 inches (50.8 mm) to about 6 inches (152.4 mm) and the diameter of the deflectable catheter shaft section 12 can range from about 5 French to about 12 French. The diameter of the deflectable catheter shaft section 12 can be about 7 French in accordance with some embodiments. Although these particular dimensions are mentioned in particular, the dimensions of the deflectable catheter shaft section 12 can vary in accordance with various applications of the deflectable catheter shaft section 12. The deflectable catheter shaft section 12 can be configured for deflection independent of the proximal catheter shaft section 20.

Tip assembly 18 comprises a tip electrode 56 having a distal end 50 and a proximal end 52. Tip electrode 56 may be configured for various functions and may include, without limitation, an active outer surface that is configured for exposure to blood and/or tissue. The tip electrode 56 may be affixed to distal end 14 of the deflectable catheter shaft section 12 in a number of ways. For instance, the tip electrode 56 may be bonded to an inner radial surface of the deflectable catheter shaft section 12 using an epoxy material. As used herein, the term "radial surface" means a surface at a radial distance from a central axis or a surface developing uniformly around a central axis (for example, but without limitation, an arcuate surface, an annular surface, or a cylindrical surface). The tip electrode 56 of the tip assembly 18 may have a recess (not shown) formed therein that is sufficiently sized and configured to receive a wire (not shown) that is connected to the tip electrode 56. One end of the wire is connected to the tip electrode 56 and the other end is connected to, for example, monitoring or recording or ablation devices, such as a radiofrequency (RF) generator. The wire is typically a pre-coated wire that is insulated from other components in the tip assembly 18. The tip electrode 56 of the tip assembly 18 may further include a recess (not shown) formed therein that is configured to receive a thermocouple (not shown). The thermocouple may be configured to measure the temperature of the tip electrode 56, targeted tissue, and/or the interface therebetween and provide feedback to the monitoring or recording or ablation devices described hereinabove. The tip electrode 56 may further include a fluid lumen configured as a passageway for irrigation fluid.

Proximal catheter shaft section 20 can also include one or more lumens (not shown). Generally, proximal catheter shaft section 20 can include a single lumen. Proximal catheter shaft section 20 can also be constructed of a series of polymer layer(s) and braid structure(s). In particular, one or more wires wound to form a cylindrical braid structure can substantially surround the one or more lumens of proximal catheter shaft section 20. In addition, a polymeric material, such as polyurethane, nylon, or various types of plastic materials such as polyether block amides offered under the trademark PEBAX^{®}, or any other suitable material, can also substantially surround the one or more lumens of proximal catheter shaft section 20. Regardless of the material used, the material must have capability to be displaced or to shrink when subjected to a process, such as for example, a heating process that is performed. The mechanical properties of the proximal catheter shaft section 20 can also be varied by varying the properties of the cylindrical braid structure(s) and the polymeric material (e.g., dimension of the cylindrical braid structure and/or durometers of the polymers). Additionally, the mechanical properties of the proximal catheter shaft section 20 can be varied along the length of the proximal catheter shaft section 20 in accordance with some embodiments of the disclosure or can be substantially constant along the entire length of the proximal catheter shaft section 20 in accordance with other embodiments of the disclosure.

The handle assembly 22 is coupled to the proximal catheter shaft section 20 at its proximal end (disposed within handle assembly 22 and not shown). The handle assembly 22 is operative to, among other things, effect movement (i.e., deflection) of the deflectable catheter shaft section 12. The handle assembly 22 includes a distal end 94 and a proximal end 96.

The catheter 10 may include any number of other elements such as, for example and without limitation, thermocouples, thermistor temperature sensors, etc. for monitoring the temperature of targeted tissue and controlling the temperature.

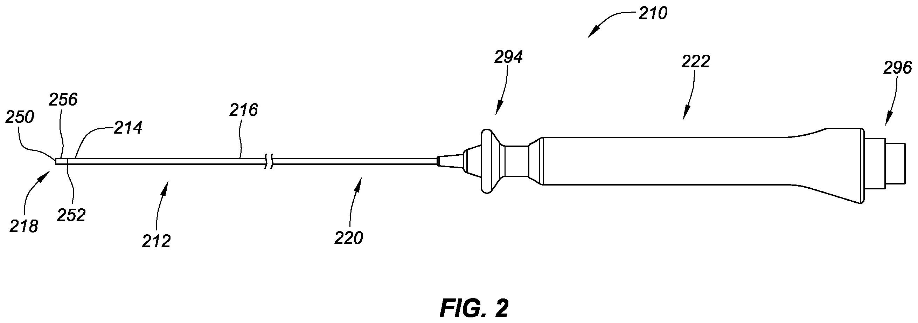

FIG. 2 generally illustrates another embodiment of a deflectable electrophysiological catheter 210. The embodiment of the catheter depicted in FIG. 2 comprises a handle assembly where a push/pull action is performed to deflect the deflectable catheter shaft section 212. The deflectable catheter shaft section 212 comprises an elongated body having a distal end 214 and a proximal end 216. The deflectable electrophysiological catheter 210 further comprises a tip assembly 218 located at the distal end of the deflectable catheter shaft section 214, a proximal catheter shaft section 220, and a handle assembly 222.

Deflectable catheter shaft section 212 is disposed between the tip assembly 218 and the proximal catheter shaft section 220. The length and diameter of the deflectable catheter shaft section 212 can vary according to the application. Generally, the length of the deflectable catheter shaft section 212 can range from about 2 inches (50.8 mm) to about 6 inches (152.4 mm) and the diameter of the deflectable catheter shaft section 212 can range from about 5 French to about 12 French. The diameter of the deflectable catheter shaft section 212 can be about 7 French in accordance with some embodiments. Although these particular dimensions are mentioned in particular, the dimensions of the deflectable catheter shaft section 212 can vary in accordance with various applications of the deflectable catheter shaft section 212. The deflectable catheter shaft section 212 can be configured for deflection independent of the proximal catheter shaft section 220.

Tip assembly 218 comprises a tip electrode 256 having a distal end 250 and a proximal end 252. Tip electrode 256 may be configured for various functions and may include, without limitation, an active outer surface that is configured for exposure to blood and/or tissue. The tip electrode 256 may be affixed to distal end 214 of the deflectable catheter shaft section 212 in a number of ways. For instance, the tip electrode 256 may be bonded to an inner radial surface of the deflectable catheter shaft section 212 using an epoxy material. As used herein, the term "radial surface" means a surface at a radial distance from a central axis or a surface developing uniformly around a central axis (for example, but without limitation, an arcuate surface, an annular surface, or a cylindrical surface). The tip electrode 256 of the tip assembly 218 may have a recess (not shown) formed therein that is sufficiently sized and configured to receive a wire (not shown) that is connected to the tip electrode 256. One end of the wire is connected to the tip electrode 256 and the other end is connected to, for example, monitoring or recording or ablation devices, such as a radiofrequency (RF) generator. The wire is typically a pre-coated wire that is insulated from other components in the tip assembly 218. The tip electrode 56 of the tip assembly 218 may further include a recess (not shown) formed therein that is configured to receive a thermocouple (not shown). The thermocouple may be configured to measure the temperature of the tip electrode 256, targeted tissue, and/or the interface therebetween and provide feedback to the monitoring or recording or ablation devices described hereinabove. The tip electrode 56 may further include a fluid lumen configured as a passageway for irrigation fluid.

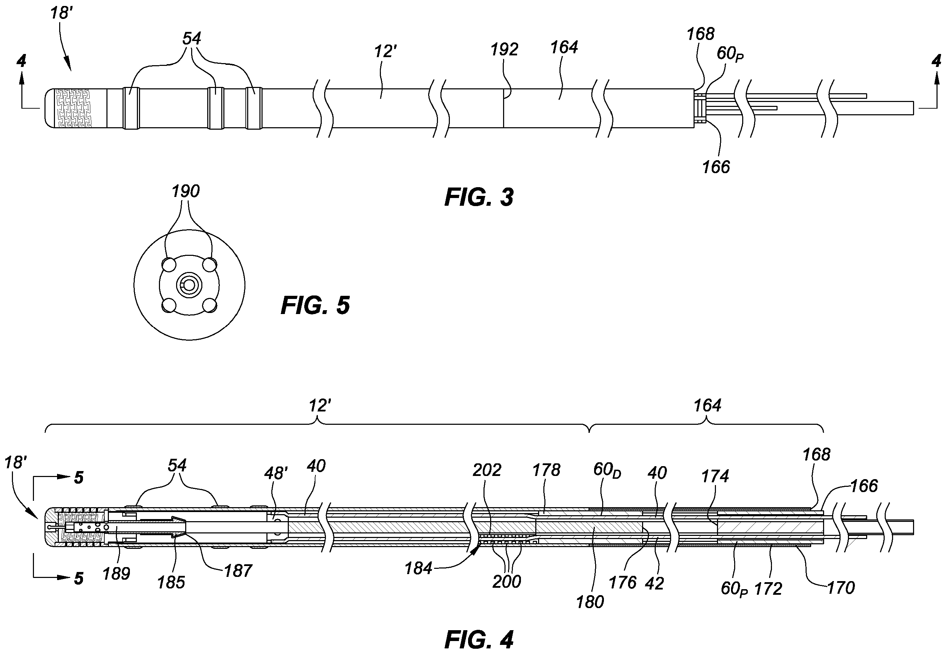

FIGS. 3 and 4 depict a deflectable catheter shaft section 12' similar to the deflectable catheter shaft section 12 shown to good advantage in, for example, FIGS. 1, and 2. As shown in FIGS. 3 and 4, the catheter shaft may include the deflectable catheter shaft section 12', an intermediate catheter shaft section 164, and a proximal catheter shaft section (not shown in FIGS. 3 and 4, but the proximal catheter shaft section, if present, would abut the right longitudinal end, as oriented in FIGS. 3 and 4, of the intermediate catheter shaft section 164). In this embodiment, two shaft couplers are used, including a proximal shaft coupler 60_{P} for coupling the proximal catheter shaft section to the intermediate catheter shaft section 164, and a distal shaft coupler 60_{D} for coupling the intermediate catheter shaft section 164 to the deflectable catheter shaft section 12'.

In at least one embodiment, the proximal catheter shaft section may comprise a portion of the handle assembly, e.g., the proximal catheter shaft section may comprise a pocket (not shown) sized and configured to receive a proximal shaft coupler 60_{P} and formed in the distal end 94 of handle assembly 22 seen in FIG. 1. In an alternative embodiment, it is possible, depending upon which handle assembly 22 is selected, that the handle assembly may connect to the proximal end 168 of the intermediate catheter section 164, or to the proximal end 166 of the proximal shaft coupler 60_{P}. In these latter configurations, the intermediate catheter shaft section 164 would be analogous to the proximal catheter section shown in, for example, FIG. 1.

Referring more particularly to FIG. 4, additional details will be described. FIG. 4 is a cross-sectional view taken along line 4-4 of FIG. 3. Starting from the right side of FIG. 4 and moving leftward, a proximal end 166 of the proximal shaft coupler 60_{P} may be seen extending proximally beyond the proximal end 168 of the intermediate catheter shaft section. It is also possible to see that the intermediate catheter shaft section 164 may include a first shaft material 170 (e.g., PEBAX) and a second shaft material 172 (e.g., PEBAX or braided mesh). A first pull wire 40 may be seen extending along the upper portion of the proximal shaft coupler 60_{P}, and a second pull 42 wire may be seen extending adjacent a lower portion of the proximal shaft coupler 60_{P}. The portion of these pull wires 40, 42 extending from the proximal end 166 of the proximal shaft coupler 60_{P} back to the handle assembly 22 (see, for example, FIG. 1) may have compression coils surrounding them. Additionally, there may be compression coils (not shown) extending between a distal end 174 of the proximal shaft coupler 60_{P} and a proximal end 176 of the distal shaft coupler 60_{D}. These compression coils would be under compression (e.g., they may be compressed 0.070 in.) to help mitigate against undesirable deformation of the intermediate catheter shaft section 164 extending between the proximal and distal shaft couplers. In the embodiment shown, the compression coils do not extend through the proximal shaft coupler, but they could in an alternative embodiment.

Moving further leftward in FIG. 4, the distal shaft coupler 60_{D}, which is depicted as joining the intermediate catheter shaft section 164 (which , as discussed above, may extend to the handle assembly 22) to the deflectable catheter shaft section 12' that extends from the distal shaft coupler to the tip assembly 18'. As shown in FIG. 4, when the first pull wire 40 exits the distal end 178 of the distal shaft coupler 60_{D}, it enters a liner 182 (e.g., a thin-walled PTFE tube). The second pull wire 42, upon exiting the distal end 178 of the distal shaft coupler 60_{D}, extends through a bendable stiffening member (e.g., a 'coil pack' or a 'spring pack' or an 'uncompacted spring pack' or a 'deflection facilitator') 184, the proximal end of which is visible in FIG. 4.

Distal to the pull ring 48' in the configuration depicted in FIGS. 3 and 4 are a plurality of ring electrodes 54 followed distally by a tip assembly 18', including, for example, a flexible tip electrode from a Therapy^{™} Cool Flex^{™} ablation catheter manufactured by St. Jude Medical, Inc. of St. Paul, Minnesota. Additional details regarding a flexible electrode tip may be found in, for example, United States patent no. US 8,187,267 B2 and United States patent application publication no. US 2010/0152731 A1. The tip assembly 18', as depicted in FIG. 4, also includes a barbed connector 185 that locks into a complementary pocket 187, thereby facilitating delivery of irrigant to a ported fluid distribution tube 189. FIG. 5 is an end view of the tip assembly 18' (looking in the direction of the arrows on line 5-5 of FIG. 4) and illustrates a plurality of irrigation ports 190 through the distal surface of the tip.

In various embodiments, a catheter may comprise a flexible tip assembly, which may be positioned and/or constructed similar to tip assemblies 18 and/or 18' described above. However, impedance values, measured with a flex tip can vary drastically and can be measured through the tip and a reference electrode located on a patient's skin. The distance between these two points and variation induced by cardiac motion and respiration can induce error into the measured values. These issues can induce errors into lesion prediction algorithms. Further, bi-pole tissue measurement between a tip electrode and a ring electrode or other adjacent electrode pairs can produce error or can lack fidelity due to relatively large gaps between the surfaces of the electrodes and the requirement of maintaining electrode tissue contact during data acquisition. As a result, ensuring that both electrodes are in contact with tissue might prove difficult and/or be inconsistent. One solution to this problem is to add a fine or small gauge electrode to the surface of an electrode or adjacent to an electrode. By adding an electrode of this type, the distance between the electrodes can be limited to the insulation thickness or distance and this can eliminate variations with measuring between the patient reference patch. Further, by decreasing the distance between the electrodes, determinations regarding lesion formation could be more quickly obtained than when using larger electrodes or those with farther space between. Further discussion of a flexible tip electrode can be found in United States Application Number 14/969,272, filed 15 December 2015, titled FLEXIBLE ELECTRODE TIP WITH HALO IRRIGATION.

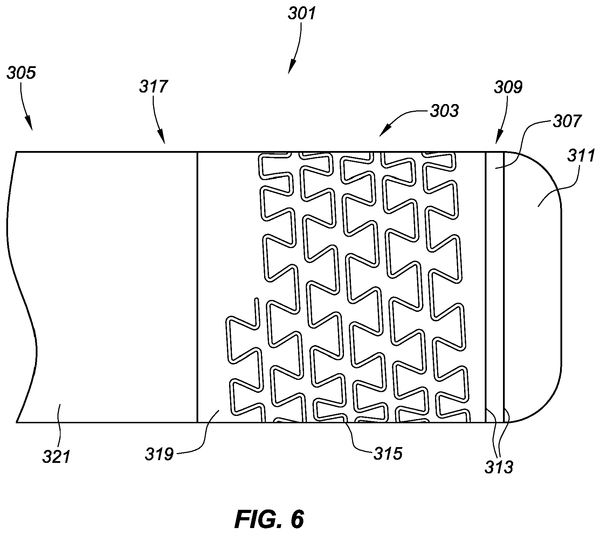

FIG. 6 illustrates a side view of a catheter 301. The catheter 301 comprises a catheter shaft 305 and a tip electrode 303. The catheter shaft 305 can comprise a catheter body 321. The catheter body 321 can comprise a catheter body distal end 317 and can be disposed proximal of the tip electrode 303. The catheter body distal end 317 can be coupled to the tip electrode 303. The catheter body 321 can be constructed of a series of polymer layer(s) and braid structure(s). In particular, one or more wires wound to form a cylindrical braid structure can substantially surround the one or more lumens of proximal catheter shaft section 20. In addition, the catheter body 321 can comprise a polymeric material, such as polyurethane, nylon, or various types of plastic materials such as polyether block amides offered under the trademark PEBAX^{®}, or any other suitable material. The tip electrode 303 can comprise an electrode wall 319, at least one slot 315, an electrode cap 311, and a wire electrode 309. The wire electrode 309 can comprise a wire 307 and an insulation 313. The insulation 313 can electrically isolate the wire 307 from rest of the tip electrode 303. In one embodiment, the insulation can comprise a polyimide. In other embodiments, the insulation can comprise other materials with insulative properties. In some embodiments, the material will be electrically and/or thermally insulative and biocompatible. The insulative material can comprise a layer that is less than half a thousandth of an inch (less than 12.7µm) in thickness. In other embodiments, the layer of insulative material can be greater than or lesser than this thickness depending on the applications that the wire electrode is designed to be used. Generally, higher voltage applications of the wire electrode require greater thicknesses of insulation. In one embodiment, the insulative material layer can comprise a thickness of one ten thousandth of an inch (2.54µm). In other embodiments, the layer of insulative material can comprise a thickness of two thousandths of an inch (50.8µm). In yet other embodiments, the thickness of the insulative material or other material can comprise ranges between these or above or below depending on the properties of the insulative material and the desired applications and procedures that will use the wire electrode.

In the illustrated embodiment, the wire electrode 309 can be disposed between the electrode wall 319 and the electrode cap 311. In other embodiments, as seen throughout the application, the wire electrode can be disposed proximal the at least one slot or in other portions of the electrodes as would be understood by one of ordinary skill in the art. The wire electrode 309 can be disposed within a tip electrode trough or tip electrode depression as described below. While the wire electrode is depicted on a flexible tip electrode, the wire electrode can be disposed on a non-flexible tip electrode, on ring electrodes, on a body of a catheter, and any other location that would be known to one of ordinary skill in the art. Further, more than one wire electrode can be present on a tip electrode, ring electrode, and/or catheter body. In some embodiments, each of the tip electrode, ring electrodes, and catheter body can comprise a plurality of wire electrodes. In other embodiments, a plurality of wire electrodes can be disposed on any one or multiple of the tip electrode, ring electrode, and catheter body. The wire electrode can comprise a bio-compatible wire. In one embodiment, the wire of the wire electrode can comprise one or more of a platinum alloy, a platinum-iridium alloy, a nickel alloy, and gold. In one embodiment, the wire of the wire electrode can comprise the same material as the surrounding tip electrode, ring electrode, or other nearby or surrounding electrode. However, in other embodiments, the wire of the wire electrode does not have to comprise the same material as the electrode tip, ring electrode, or other material present on or within the catheter.

The wire electrode can be used to predict lesion formation. The wire electrode can be used or configured to determine or sense an impedance of tissue that is contacting the wire electrode. Changes in the impedance can be used to determine contact with a tissue. Further, spectral scans can be performed to determine tissue depth.

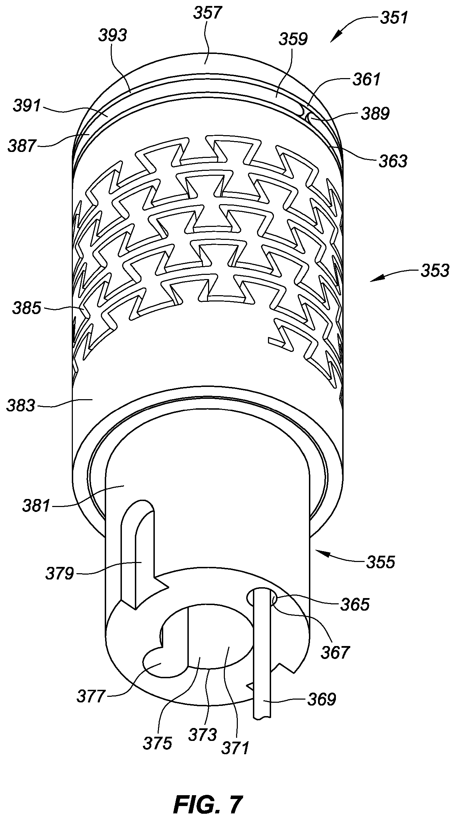

FIG. 7 illustrates an isometric side view of a tip assembly 351. The tip assembly 351 comprises an electrode stem 355 and a tip electrode 353. The electrode stem 355 comprises an anchor point 379, a stem lumen 373, a stem wire lumen 365, and a stem outer wall 381. The stem outer wall 381 can define an outer diameter of the electrode stem 355. Further, the stem outer wall 381 can be configured to be placed within a distal end of a catheter body and can be secured and/or coupled to the distal end of the catheter body. The anchor point 379 can comprise a depression within the stem outer wall 381. The anchor point 379 can be sized and shaped to contain an anchor for the tip assembly 351 and can extend from a proximal end of the electrode stem 355 to a more distal portion. In one embodiment, the anchor point can extend from a proximal end of the electrode stem to a distal portion of the electrode stem. In another embodiment, the anchor point can extend from a proximal end of the electrode stem to a middle portion of the electrode stem. In another embodiment, the anchor point can comprise a first anchor point and electrode stem can comprise a second anchor point. The stem lumen 373 can comprise a lumen wall 375. The lumen wall 375 can define the stem lumen 373. In the illustrated embodiment, the lumen wall 375 can define a stem major lumen 371 and a stem minor lumen 377 within the stem lumen 373. The stem lumen 373 can extend from a proximal end of the electrode stem 355 to a distal end of the stem lumen 373. In the illustrated embodiment, the stem lumen can comprise a figure-eight shape. In the illustrated embodiment, the stem major lumen 371 and the stem minor lumen 377 can be coupled or conjoined. Further, in the illustrated embodiment the stem minor lumen 377 can comprise a smaller diameter than the stem major lumen 371. In other embodiments, the stem minor lumen can comprise a diameter that is the same or similar size to the stem major lumen. Further, in other embodiments, the stem minor lumen and stem major lumen can be separate and not conjoined. The stem wire lumen 365 can comprise a wire lumen wall 367. The wire lumen wall 367 can define the stem wire lumen 365. The stem wire lumen 365 can extend from a proximal end of the electrode stem 355 to a distal end of the stem lumen 373. The tip electrode 353 can comprise an electrode cap 357, a wire electrode 387, an electrode wall 383, and at least one slot 385. The wire electrode 387 can comprise a wire 391, an insulation 393, and a wire electrode end 389. The insulation 393 can surround a portion of the wire 391 as described throughout this application and can be used to electrically insulate the wire from the rest of the tip electrode. The wire electrode end can comprise an end portion of the wire electrode. The wire electrode can pass from an inner portion of the tip electrode and be disposed within a channel or depression within an outer surface of the tip electrode. The wire electrode can be disposed within the channel or depression as it wraps around an outer circumference of the tip electrode. When the distal end or distal portion of the wire electrode meets with the portion of the wire electrode that exits the interior portion of the tip electrode, the distal end or portion of the wire electrode can be laser welded to secure the distal end or portion in place. In another embodiment, the distal end of the wire electrode can be laser welded to a more proximal portion of the wire electrode. In this embodiment, the wire electrode can then be laser welded to the tip electrode. In yet another embodiment, the wire electrode can be laser welded to a ring electrode. In yet another embodiment, a distal portion of the wire electrode can be secured within an inner portion of the tip electrode or catheter body. In other embodiments, other methods can be used to secure the wire electrode and/or the distal end or end portion of the wire electrode to the tip electrode. In one embodiment, a glue or other adhesive can be used to secure the wire electrode to the tip electrode. In yet another embodiment, the wire electrode can be swaged to secure the electrode. In some embodiments, when the wire electrode is initially secured to the tip electrode or catheter body, the insulation of the wire electrode can completely or mostly surround the wire of the wire electrode. In some embodiments, the wire can comprise a circular cross-section. The insulation and wire that protrudes above an outer surface of the tip electrode or catheter body can then be removed so that a flat outer surface is created between an outer surface of the electrode, wire electrode insulation and wire electrode wire. By removing the portion of the wire electrode that protrudes above the height of neighboring surfaces a smooth outer surface can be created while keeping a layer of insulation between the wire electrode and any adjacent surfaces. In one embodiment, the portion of the wire electrode protruding beyond an outer surface of an adjacent electrode can be ground to product the exposed electrode surface. Grinding the electrode to expose the electrode surface can have the advantage of ensuring virgin metal as adhesive or other products can cause issues with signal acquisition if disposed on a surface of the wire electrode. In yet another embodiment, the wire electrode be sized and shaped to fit within the channel and not require additional processing of the height of the electrode to create the wire electrode described herein. In some embodiments, a thermal sensor can be placed adjacent and/or underneath the wire electrode to determine local temperatures.

The wire electrode can be advantageous as it allows for an inexpensive way to add an additional electrode adjacent and/or within other electrodes. Further, the wire electrode can alleviate concerns regarding orientation of the device as in some embodiments the wire electrode surrounds an outer circumference of the tip electrode and/or catheter shaft. Additionally, the wire electrode can allow for a "ring 2" to be close to the lesion itself. In one embodiment, as illustrated herein, the wire electrode can be disposed adjacent the tip of the tip electrode. In another embodiment, the wire electrode can be disposed around 0.6 mm from distal end of the tip electrode. Further, the wire electrode can be used to acquire improved data for bipolar measurements from the medical device.

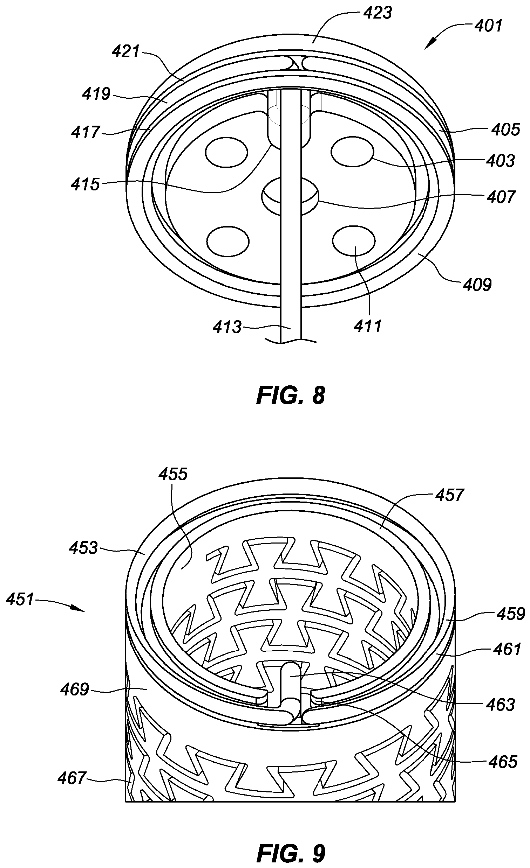

FIG. 8 depicts a side view of one embodiment of an electrode cap 401. The electrode cap 401 can comprise a cap outer surface 423, a center irrigation port 407, at least one outer irrigation port 411, a wire electrode lumen 415, a proximal end 409, and a wire electrode 405. The wire electrode 405 can comprise a wire 419, an insulation 417, and a wire electrode conductor 413. The wire electrode conductor 413 can be coupled to the wire 419. In one embodiment, the wire electrode conductor 413 can be contiguous with the wire 419 and they can comprise a single structure. Further, a portion of the wire electrode conductor 413 can be disposed within the wire electrode lumen 415. The insulation 417 can electrically insulate the wire 419 from the cap outer surface 423 and from the proximal end 409. In the illustrated embodiment, the wire electrode 405 is disposed within the electrode cap 401. The center irrigation port 407 and the at least one outer irrigation port 411 can comprise through-holes to allow for irrigant to pass from the proximal end 409 of the electrode cap to the distal end of the electrode cap 401. Further, the center irrigation port 407 and the at least one outer irrigation port 411 can be configured to allow for irrigant to pass from an inner portion of the tip electrode to an area outside and distal of the electrode cap and the tip electrode. The proximal end 409 of the electrode cap 401 can be shaped to couple to a distal end of an electrode wall.

FIG. 9 illustrates a side view of one embodiment of a partial tip electrode 451. The partial tip electrode 451 can comprise a distal end 457, a sidewall 469, an inner wall 455, at least one slot 467, and a wire electrode 453. The wire electrode 453 can comprise a wire 461, an insulation 459, and a wire electrode conductor 463. The wire electrode 453 can be disposed distal of the sidewall 469. In the illustrated embodiment, the wire electrode 453 can be coupled to the inner wall 455. The inner wall 455 can comprise an opening 465. The wire electrode conductor 463 can pass through the opening 465 of the inner wall 455 to couple to the wire electrode 453. The insulation 459 can surround an interior facing portion of the wire 461 as described throughout the application. In the illustrated embodiment, the sidewall 469, the inner wall 455, and the wire electrode 453 can comprise a circular cross-section. Further, in the illustrated embodiment, an outer diameter of the wire electrode 453 can be the same as an outer diameter of the sidewall 469. In other embodiments, the outer diameter of the wire electrode 453 can be the less than the outer diameter of the sidewall 469. In yet other embodiments, the outer diameter of the wire electrode 453 can be the greater than the outer diameter of the sidewall 469.

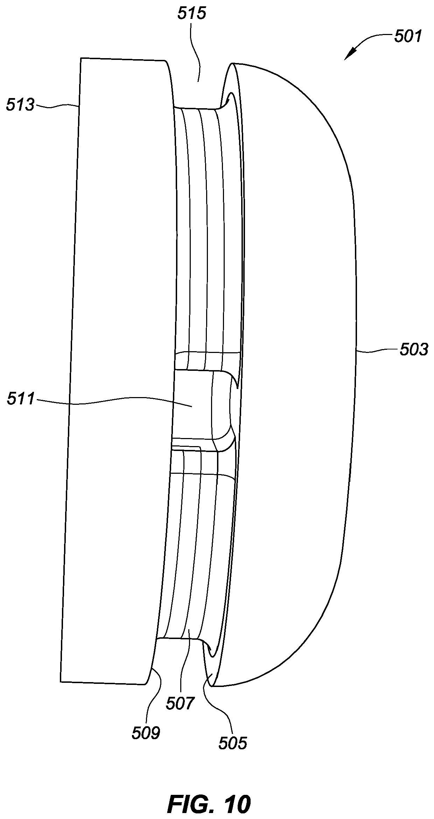

FIG. 10 illustrates a side view of one embodiment of an electrode cap 501. The electrode cap 501 can comprise a cap outer surface 503, a channel 515, a cap opening 511, and a proximal end 513. The channel 515 can be defined by a channel proximal wall 509, a channel inner wall 507, and a channel distal wall 505. The channel 515 can be sized and shaped to contain a wire electrode as described herein. The cap opening 511 can allow for a wire electrode conductor to pass from an inner portion of the electrode cap and couple to a wire electrode. In the illustrated embodiment, the channel inner wall 507 can be coupled to the channel distal wall 505 and the channel proximal wall 509. The channel inner wall 507 can comprise a flared portion where the channel inner wall 507 couples to the channel proximal wall 509 and to the channel distal wall 505.

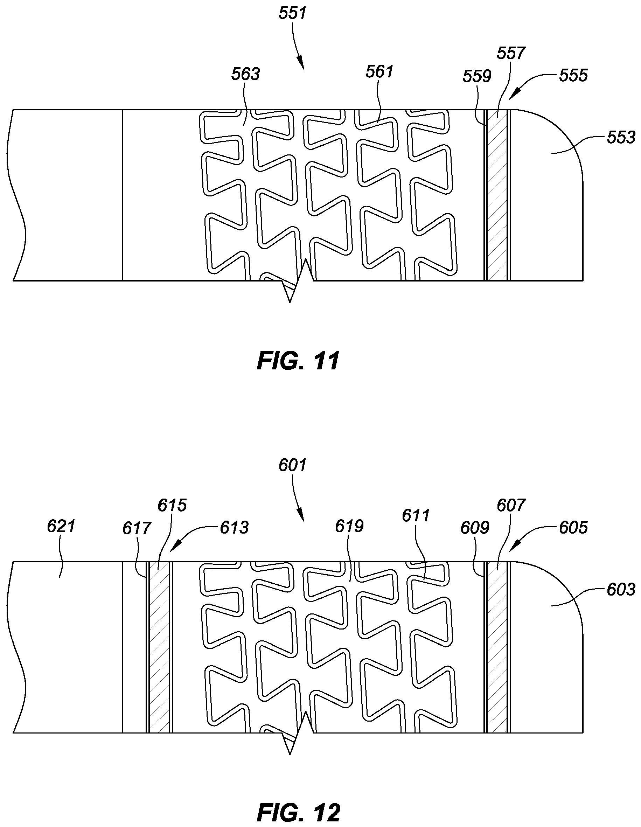

FIG. 11 illustrates a partial side view of a distal portion of a tip electrode 55 1The tip electrode 551 can comprise an electrode cap 553, a sidewall 563, at least one slot 531, and a wire electrode 555. The wire electrode 555 can comprise a wire 557 and an insulation 559. In the illustrated embodiment, the wire electrode 555 can be disposed around an outer circumference of the sidewall 563. The wire electrode 555 can further be disposed at a distal end of the sidewall 563 and adjacent to the electrode cap 553. The insulation 559 can cover an interior portion of the wire 557. The insulation 559 can electrically insulate the wire 557 from the sidewall 563 and the electrode cap 553.

FIG. 12 illustrates a partial side view of a tip electrode 601. The tip electrode 601 can be coupled to a catheter body 621. The tip electrode 601 can comprise an electrode cap 603, a sidewall 619, at least one slot 611, a first wire electrode 605, and a second wire electrode 613. The first wire electrode 605 can comprise a first wire 607 and a first insulation 609. The second wire electrode 613 can comprise a second wire 615 and a second insulation 617. In the illustrated embodiment, the first wire electrode 605 can be disposed around an outer circumference of the sidewall 619. The first wire electrode 605 can further be disposed at a distal end of the sidewall 619 and adjacent to the electrode cap 603. The first insulation 609 can cover an interior portion of the first wire 607. The first insulation 609 can electrically insulate the first wire 607 from the sidewall 619 and the electrode cap 603 The second wire electrode 613 can be disposed around an outer circumference of the sidewall 619. The second wire electrode 613 can further be disposed at a proximal end of the sidewall 619 and adjacent to the catheter body 621. The second insulation 617 can cover an interior portion of the second wire 615. The second insulation 617 can electrically insulate the second wire 615 from the sidewall 619. In one embodiment, the second wire electrode 613 can be disposed next to the catheter body 621. In another embodiment, a proximal portion of the sidewall can be positioned between the second wire electrode and the catheter body. In yet another embodiment, at least a portion of the at least one slot can be positioned proximal of the second wire electrode.

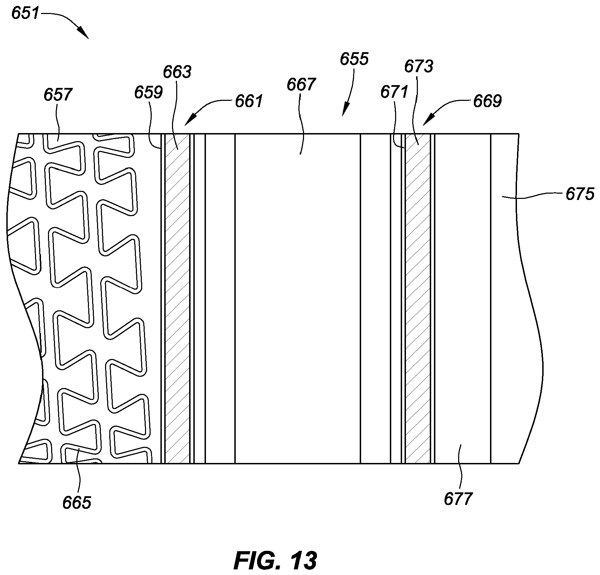

FIG. 13 depicts a partial side view of a catheter 651. The catheter 651 can comprise a catheter shaft 655 and a tip electrode 653. The tip electrode 653 can comprise a sidewall 657, at least one slot 665, and a first wire electrode 661. The first wire electrode 661 can comprise a first wire 663 and a first insulation 659. The catheter shaft 655 can comprise a shaft body 677, a first ring electrode 667, a second ring electrode 675, and a second wire electrode 669. The second wire electrode 669 can comprise a second wire 673 and a second insulation 671. In the illustrated embodiment, the first ring electrode 667 can be disposed adjacent a proximal end of the tip electrode 653. A portion of the shaft body 677 can be disposed between the first ring electrode 667 and the tip electrode 653 This portion of the shaft body 677 can electrically insulate the first ring electrode 667 from the tip electrode 653. In the illustrated embodiment, the second wire electrode 669 can be disposed on an exterior portion of the shaft body proximal of the first ring electrode 667. In one embodiment, the second wire electrode can be evenly spaced between the first ring electrode and the second ring electrode. In another embodiment, the second wire electrode can be spaced adjacent the first ring electrode and spaced farther from the second ring electrode. In yet another embodiment, the second wire electrode can be placed adjacent the second ring electrode and spaced farther from the first ring electrode. In yet another embodiment, the second wire electrode can be disposed proximal of the second ring electrode. In the illustrated embodiment, the second ring electrode can be disposed proximal of the first ring electrode and the second wire electrode. The second ring electrode can be electrically insulated from the second wire electrode.

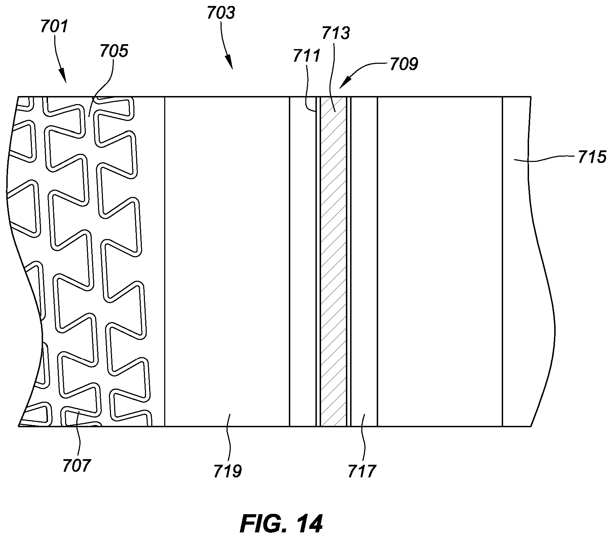

FIG. 14 depicts a partial side view of a catheter. The catheter can comprise a catheter shaft 703 and a tip electrode 701. The tip electrode 701 can comprise a sidewall 705 and at least one slot 707. The catheter shaft 703 can comprise a shaft body 719, a first ring electrode 717, a second ring electrode 715 and a first wire electrode 709. The first wire electrode 709 can comprise a first wire 713 and a first insulation 711. In the illustrated embodiment, the first wire electrode 709 can be disposed within the first ring electrode 717. In one embodiment, the first wire electrode can be disposed around the entire outer circumference of the first ring electrode. In another embodiment, the first wire electrode can be disposed around a portion of the outer circumference of the first ring electrode. In yet another embodiment, the first wire electrode can be a longitudinally extending electrode. In this embodiment, a proximal end of the first wire electrode can be disposed in a proximal portion of the first ring electrode. A distal portion of the longitudinally extending first wire electrode can be disposed in a proximal portion of the first ring electrode. In yet another embodiment, the first wire electrode can comprise a longitudinal electrode that passes through the first ring electrode. In this embodiment, a proximal end of the first wire electrode can be disposed proximal a proximal end of a proximal end of the first ring electrode. A distal portion of the longitudinally extending first wire electrode can be disposed distal of a distal end of the first ring electrode. In yet another embodiment, the catheter can further comprise a second wire electrode. The second wire electrode can be disposed on one of the tip electrode, the catheter body, the first ring electrode, and/or the second ring electrode. In another embodiment, the catheter can comprise a plurality of wire electrodes. In one embodiment, a plurality of wire electrodes can be disposed on a single or multiple ring electrodes. In one embodiment, a plurality of longitudinally extending wire electrodes can be disposed on a ring electrode.

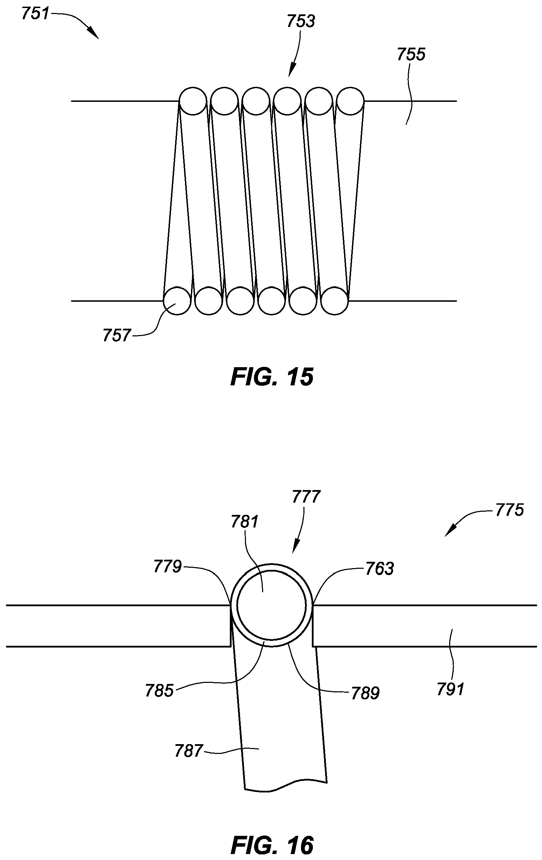

FIG. 15 depicts a side view of another embodiment of a wire electrode 753. The wire electrode 753 can be disposed on a catheter shaft 751. The catheter shaft 751 can comprise a shaft body 755 and a wire electrode 753. In the illustrated embodiment, the wire electrode 753 can comprise a wire 757 and an insulation (not shown). The wire can wrap around an exterior surface of the shaft body multiple times. In one embodiment, the wire electrode can form a coil around an outer surface of the shaft body. In one embodiment, a single loop of the wire of the wire electrode can be in contact with other loops of the wire that are located proximal or distal of the single loop of wire along the shaft body. In another embodiment, a single loop of the wire of the wire electrode can be spaced from other loops of the wire electrode that are located proximal or distal of the single loop of wire along the shaft body. The wire electrode depicted in FIG. 15 can be wound around the shaft body a single time, two times, three times, or more. The number of tunings of the wire electrode and the spacing between the loops of the wire electrode can vary depending on the design of the catheter. The longitudinal length of the wire electrode, the number of loops in the electrode, and the spacing between adjacent loops of the electrode can be changed depending on the desired flexibility of the catheter shaft, the energy being delivered or received to the wire electrode, or for other circumstances as would be known to one of ordinary skill in the art. Further, in some embodiments, the wire electrode can comprise a wire that is free of insulation in a portion of the electrode that is wrapped around the catheter shaft. In this embodiment, an interior portion of the wire electrode can comprise an insulation that can electrically and/or thermally insulate an interior portion of the wire electrode from other components within the shaft body. In one embodiment, an portion of the wire electrode within the shaft body can comprise a wire conductor, as described throughout this application.

FIG. 16 depicts a close-up cross-sectional view of a catheter shaft 775. The catheter shaft 775 can comprise a shaft body 791 and a wire electrode 777. The wire electrode 777 can comprise a wire 781, an insulation 785, and a wire conductor 787. The shaft body 791 can comprise a proximal wall 783 and a distal wall 779. The proximal wall 783 and the distal wall 779 can define a through hole 789. In one embodiment, the through hole can comprise a circular cut-out bounded by the shaft body. In other embodiments, the through hole can comprise other shaped voids within the shaft body. The through hole 789 can be defined in a size and shape to allow the wire electrode 777 and the insulation 785 to pass from an interior portion of the catheter shaft 795 to an exterior portion of the catheter shaft 795. In one embodiment, after passing from an interior portion of the catheter shaft 795, the wire electrode 777 can be disposed within a groove within the shaft body 791 as described throughout the application. After being coupled to the groove or channel as described herein, the wire electrode can be ground or otherwise processed to reduce the wire electrode to a height of the surrounding surface. In the illustrated embodiment, the wire conductor 787 can be coupled to the wire 781. The wire conductor 787 can be used and/or configured to deliver electrical signals to and/or from the wire 781 of the wire electrode 777 to a system or connector external of the catheter shaft 775. In one embodiment, the wire conductor can comprise an insulation to insulate any signals being transferred by the wire conductor from outside interference or from interfering with other components within the catheter shaft. In one embodiment, the wire conductor can comprise the same material as the wire. In other embodiments, the wire conductor can comprise the same material as the wire, but can comprise a different shape. In yet another embodiment, the wire conductor can comprise a different material than the wire and can be the same or different shape than the wire. In some embodiments, the wire conductor can be integral to the wire. In yet other embodiments, the wire conductor can be coupled to the wire through a conductive glue, bonded, welded, soldered, fused, or otherwise secured.

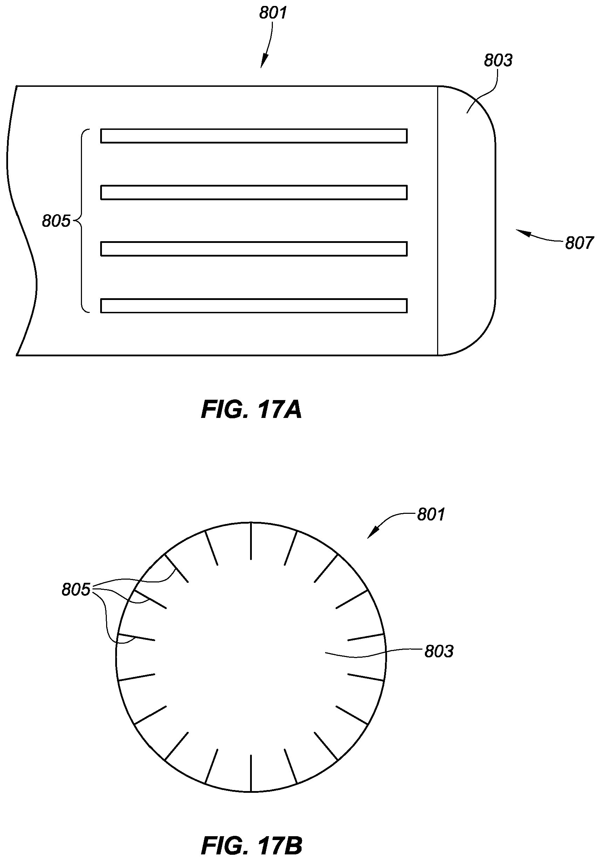

FIGS. 17A and 17B depict a side view and an end view of one embodiment of a tip electrode 801. The tip electrode 801 can comprise a distal end 807, a tip surface 803, and a plurality of wire electrodes 805. The plurality of wire electrodes 805 can each run longitudinally from a proximal portion of the tip electrode 801 to a distal portion of the tip electrode 801. In one embodiment, each of the plurality of wire electrodes 805 can be evenly spaced from each of the other wire electrodes around an outer circumference of the tip electrode 801. In one embodiment, each of the plurality of wire electrodes can individually receive or deliver energy, independent of any neighboring wire electrodes. In another embodiment, energy can be delivered or received from a subset or all of the plurality of wire electrodes as a group. In the illustrated embodiment, each of the plurality of wire electrodes terminates prior to a distal end of the tip electrode. In one embodiment, one or more of the plurality of wire electrodes can terminate at the distal tip. In another embodiment, at least one of the plurality of wire electrodes can extend pass the distal end of the tip electrode and extend to the opposite side of the tip electrode.

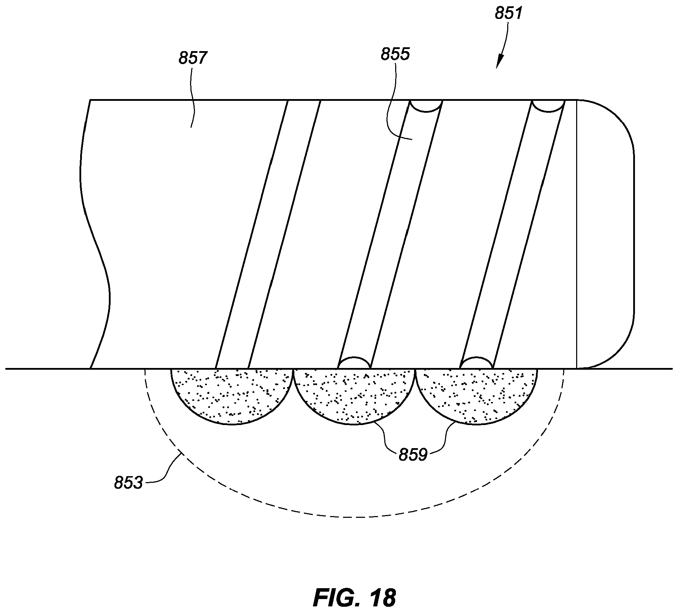

FIG. 18 depicts a side view of a tip electrode 851 contacting a tissue 853. The tip electrode 851 can comprise a tip surface 857 and a wire electrode 855. The wire electrode 855 can comprise a helical pattern around the tip electrode 851. The tissue 853 can comprise a plurality of lesions 859. The plurality of lesions 859 can be created when the tip electrode 851 contacts the tissue 853 and energy is delivered by the wire electrode 855. In one embodiment, the wire electrode can comprise a plurality of wire electrodes. In one embodiment, an individual wire electrode can comprise one turn on the tip electrode. The tip electrode can comprise multiple wire electrodes separated from each other. In this embodiment, energy can be passed between two wire electrodes. When energy is passed between two of the wire electrodes on the tip electrode, lesion formation within the tissue can be controlled. In another embodiment, energy can be delivered by one or more of the wire electrodes and received by the tip surface of the tip electrode. In another embodiment, energy can be delivered by the tip surface of the tip electrode and received by one or more of the wire electrodes. As the wire electrode is electrically insulated from the tip surface, the energy delivered passes through the tissue before being received by a separate component of the tip electrode or external device.

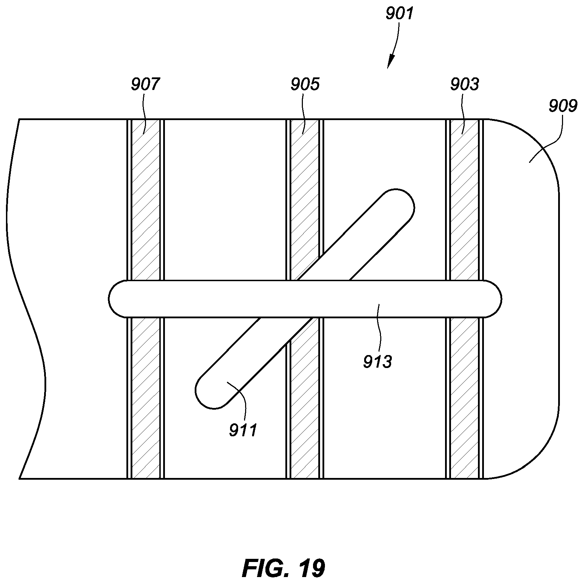

FIG. 19 depicts a side view of a tip electrode 901. The tip electrode 901 comprises a tip surface 909, a first axial wire electrode 903, a second axial wire electrode 905, a third axial wire electrode 907, a longitudinal wire electrode 913, and a slanted wire electrode 911. As shown in the illustrated embodiment, a tip electrode according to the disclosure can comprise multiple wire electrodes of differing lengths and orientations. Further, different wire electrodes can overlap one another and be electrically insulated from the other wire electrodes and the tip surface. While the illustrated embodiment depicts a set of electrodes, various orientations, lengths, and wire sizes can be incorporated into a single tip electrode using the wire electrodes described throughout this application.

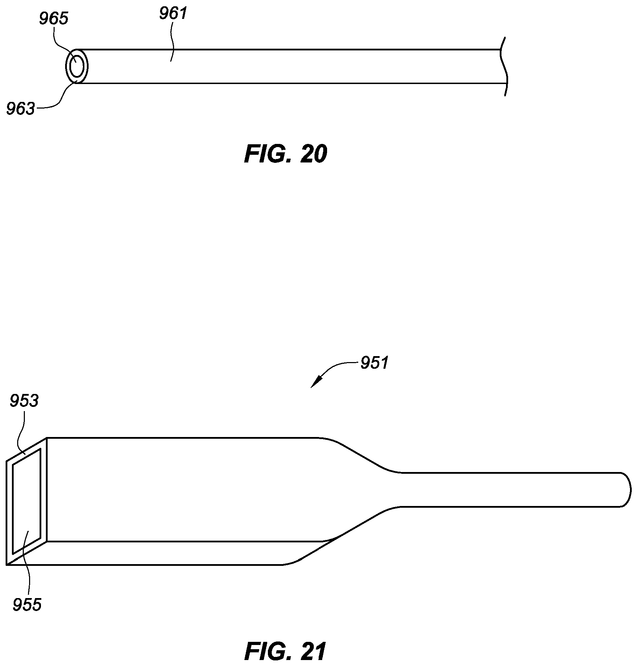

FIG. 20 depicts an isometric side view of one embodiment of a wire electrode 961. The wire electrode 961 can comprise a wire 965 and an insulation 963. In the illustrated embodiment, the wire electrode can comprise a circular cross section. The wire electrode can be used in a variety of uses as described throughout this application. Further, a proximal portion of the wire electrode can comprise a wire electrode conductor as described herein.

FIG. 21 depicts an isometric side view of another embodiment of a wire electrode 951. The wire electrode 951 can comprise a wire 955 and an insulation 953. In the illustrated embodiment, the wire electrode can comprise a rectangular cross section. In this embodiment, a first set of opposing sides can be longer than a second set of opposing sides. The wire electrode can be used in a variety of uses as described throughout this application. Further, a proximal portion of the wire electrode can comprise a wire electrode conductor as described herein.

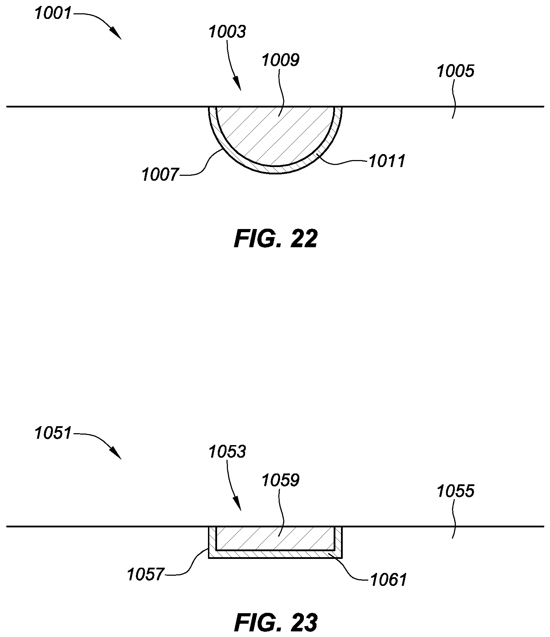

FIG. 22 depicts a close-up, cross-sectional view of an electrode 1001. The electrode 1001comprises an electrode outer surface 1005, a channel 1007, and a wire electrode 1003. The wire electrode 1003 can comprise a wire 1009 and an insulation 1011. The channel 1007 can comprise a depression within the electrode outer surface 1005. The channel 1007 can be sized and shaped to fit a portion of the wire electrode 1003 within the channel 1007. In the illustrated embodiment, the channel 1007 can comprise a hemi-spherical shape. The insulation 1011 of the wire electrode 1003 can electrically isolate the wire 1009 from the electrode outer surface 1005.

FIG. 23 depicts a close-up, cross-sectional view of an electrode 1051. The electrode 1051 comprises an electrode outer surface 1055, a channel 1057, and a wire electrode 1053. The wire electrode 1053 can comprise a wire 1059 and an insulation 1061. The channel 1057 can comprise a depression within the electrode outer surface 1055. The channel 1057 can be sized and shaped to fit a portion of the wire electrode 1053 within the channel 1057. In the illustrated embodiment, the channel 1057 can comprise a rectangular shape. The insulation 1061 of the wire electrode 1053 can electrically isolate the wire 1059 from the electrode outer surface 1055.

While a hemi-spherical and rectangular shape of a channel are illustrated in FIGS. 22 and 23, other shapes can be provided for placement of a wire electrode. Further, other portions of a catheter can comprise channels for containing a wire electrode. In various embodiments, the tip electrode, ring electrodes, catheter shaft, or other portions of a catheter can comprise channels for wire electrodes. In one embodiment, the channel can be created by drilling, sanding, or otherwise removing material from a surface of an electrode to create a channel. In another embodiment, a channel can be created when the tip is initially formed. In this instance a mold or other device can be used to form the channel. In one embodiment, when the wire electrode is disposed within a catheter shaft, the channel can be micro-molded by the wire electrode. In yet other embodiments, the channel can be formed through reflowing a catheter shaft after placement of the wire electrode. In one embodiment, the wire can be ground or otherwise processed to bring the height of the wire electrode to the level of the surrounding catheter shaft.

Although at least one embodiment of a tip electrode has been described above with a certain degree of particularity, those skilled in the art could make numerous alterations to the disclosed embodiments without departing from the scope of this disclosure. All directional references (e.g., upper, lower, upward, downward, left, right, leftward, rightward, top, bottom, above, below, vertical, horizontal, clockwise, and counterclockwise) are only used for identification purposes to aid the reader's understanding of the present disclosure, and do not create limitations, particularly as to the position, orientation, or use of the devices. Joinder references (e.g., affixed, attached, coupled, connected, and the like) are to be construed broadly and can include intermediate members between a connection of elements and relative movement between elements. As such, joinder references do not necessarily infer that two elements are directly connected and in fixed relationship to each other. It is intended that all matter contained in the above description or shown in the accompanying drawings shall be interpreted as illustrative only and not limiting. Changes in detail or structure can be made without departing from the scope of the disclosure as defined in the appended claims.

## Claims

1. A catheter tip electrode (601), comprising:
a flexible electrode structure configured to flex upon application of an external force;
a distal end portion adjacent the flexible electrode structure and defining a distal end;
and
a first wire electrode (605; ), wherein the first wire electrode comprises a wire (607;) and an insulation (609), and said first wire electrode is electrically insulated from the flexible electrode structure, wherein the insulation comprises an insulative material layer;
a second wire electrode (613), wherein the second wire electrode comprises a second wire (615) and an insulation (617), and said second wire electrode is electrically insulated from the flexible electrode structure, wherein the insulation comprises an insulative material layer;
wherein the first wire electrode (605) and the second wire (613) electrode each wrap around an outer circumference of the flexible electrode structure, wherein the first wire electrode (605) is disposed adjacent the distal end and the second wire electrode (613) is disposed on a proximal portion of the flexible electrode structure.

2. A catheter assembly comprising:
a catheter body (621);
the tip electrode (601) according to claim 13,
wherein the tip electrode (601) is coupled to a distal end of the catheter body (621).

3. The catheter assembly according to claim 2, wherein the catheter assembly further comprises a third wire electrode (669).

4. The catheter assembly according to claim 3, wherein the first wire electrode (605) is disposed on the tip electrode (601) and wherein the third wire electrode (669) is disposed on the catheter body (621).

5. The catheter assembly according to claim 4, wherein the third wire electrode (669) extends around an outer circumference of the catheter body (621).

6. The catheter tip electrode according to claim 1 or the catheter assembly of any of claims 2 to 5 wherein the insulation of the first and/or second wire electrode comprises an insulative material layer that is less than 0.0508mm thick, optionally 0.0127mm to 0.0508mm thick, 0.0508mm thick, 0.0254mm thick, preferably less than 0.0127mm.

## Patentansprüche

1. Katheterspitzenelektrode (601), umfassend:
eine flexible Elektrodenstruktur, die dazu konfiguriert ist, sich bei Anwendung einer externen Kraft zu biegen;
einen distalen Endabschnitt, der an die flexible Elektrodenstruktur angrenzt und ein distales Ende definiert;
und
eine erste Drahtelektrode (605), wobei die erste Drahtelektrode einen Draht (607) und eine Isolierung (609) umfasst und die besagte erste Drahtelektrode von der flexiblen Elektrodenstruktur elektrisch isoliert ist, wobei die Isolierung eine Isoliermaterialschicht umfasst;
eine zweite Drahtelektrode (613), wobei die zweite Drahtelektrode einen zweiten Draht (615) und eine Isolierung (617) umfasst und die besagte zweite Drahtelektrode von der flexiblen Elektrodenstruktur elektrisch isoliert ist, wobei die Isolierung eine Isoliermaterialschicht umfasst;
wobei sich die erste Drahtelektrode (605) und die zweite Drahtelektrode (613) jeweils um einen Außenumfang der flexiblen Elektrodenstruktur wickeln, wobei die erste Drahtelektrode (605) angrenzend an das distale Ende angeordnet wird und die zweite Drahtelektrode (613) auf einem proximalen Abschnitt der flexiblen Elektrodenstruktur angeordnet wird.

2. Katheteranordnung, umfassend:
einen Katheterkörper (621);
die Spitzenelektrode (601) nach Anspruch 13,
wobei die Spitzenelektrode (601) an ein distales Ende des Katheterkörpers (621) gekoppelt ist.

3. Katheteranordnung nach Anspruch 2, wobei die Katheteranordnung ferner eine dritte Drahtelektrode (669) umfasst.

4. Katheteranordnung nach Anspruch 3, wobei die erste Drahtelektrode (605) auf der Spitzenelektrode (601) angeordnet wird und wobei die dritte Drahtelektrode (669) auf dem Katheterkörper (621) angeordnet wird.

5. Katheteranordnung nach Anspruch 4, wobei sich die dritte Drahtelektrode (669) um einen Außenumfang des Katheterkörpers (621) erstreckt.

6. Katheterspitzenelektrode nach Anspruch 1 oder Katheteranordnung nach einem der Ansprüche 2 bis 5, wobei die Isolierung der ersten und/oder der zweiten Drahtelektrode eine Isoliermaterialschicht umfasst, die weniger als 0,0508 mm dick, optional 0,0127 mm bis 0,0508 mm dick, 0,0508 mm dick, 0,0254 mm dick, vorzugsweise weniger als 0,0127 mm ist.

## Revendications

1. Électrode de pointe de cathéter (601), comprenant :
une structure d'électrode flexible configurée pour fléchir lors de l'application d'une force externe ;
une partie d'extrémité distale adjacente à la structure d'électrode flexible et définissant une extrémité distale ;
et
un premier fil-électrode (605), dans laquelle le premier fil-électrode comprend un fil (607) et une isolation (609), et ledit premier fil-électrode est isolé électriquement de la structure d'électrode flexible, dans laquelle l'isolation comprend une couche de matériau isolant ;
un deuxième fil-électrode (613), dans laquelle le deuxième fil-électrode comprend un deuxième fil (615) et une isolation (617), et ledit deuxième fil-électrode est isolé électriquement de la structure d'électrode flexible, dans laquelle l'isolation comprend une couche de matériau isolant ;
dans laquelle le premier fil-électrode (605) et le deuxième fil-électrode (613) s'enroulent chacun autour d'une circonférence externe de la structure d'électrode flexible, dans laquelle le premier fil-électrode (605) est disposé adjacent à l'extrémité distale et le deuxième fil-électrode (613) est disposé sur une partie proximale de la structure d'électrode flexible.

2. Ensemble de cathéter comprenant :
un corps de cathéter (621) ;
l'électrode de pointe (601) selon la revendication 13,
dans lequel l'électrode de pointe (601) est couplée à une extrémité distale du corps de cathéter (621),

3. Ensemble de cathéter selon la revendication 2, dans lequel l'ensemble de cathéter comprend en outre un troisième fil-électrode (669).

4. Ensemble de cathéter selon la revendication 3, dans lequel le premier fil-électrode (605) est disposé sur l'électrode de pointe (601) et dans lequel le troisième fil-électrode (669) est disposé sur le corps de cathéter (621).

5. Ensemble de cathéter selon la revendication 4, dans lequel le troisième fil-électrode (669) s'étend autour d'une circonférence externe du corps de cathéter (621).

6. Électrode de pointe de cathéter selon la revendication 1 ou ensemble de cathéter selon l'une quelconque des revendications 2 à 5, dans lequel l'isolation du premier et/ou du deuxième fil-électrode comprend une couche de matériau isolant qui mesure moins de 0,0508 mm d'épaisseur, facultativement de 0,0127 mm à 0,0508 mm d'épaisseur, 0,0508 mm d'épaisseur, 0,0254 mm d'épaisseur, de préférence moins de 0,0127 mm.
